# EUROPÄISCHE PATENTSCHRIFT

(11) **EP 0 479 877 B1**
(45) Veröffentlichungstag und Bekanntmachung des Hinweises auf die Patenterteilung: **12.10.1994**
(21) Anmeldenummer: 90910680.9
(22) Anmeldetag: 25.06.1990
(51) Int. Cl.: C07C 209/26

(54) **VERFAHREN ZUR HERSTELLUNG VON N-ALKYL-HALOGENANILINEN**
METHOD OF PREPARING N-ALKYL-HALOANILINES
PROCEDE DE PRODUCTION DE N-ALKYLANILINES HALOGENES

(30) Priorität: 30.06.1989 DE 3921447
(43) Veröffentlichungstag der Anmeldung: 15.04.1992
(73) Patentinhaber: HOECHST AKTIENGESELLSCHAFT, 65926 Frankfurt am Main (DE)
(72) Erfinder: BRODT, Werner, D-6239 Eppstein/Taunus (DE); PAPENFUHS, Theodor, D-6000 Frankfurt am Main (DE)
(86) Internationale Anmeldenummer: EP9001003
(87) Internationale Veröffentlichungsnummer: WO9100262

(56) Entgegenhaltungen:
- DE-A- 2 941 070
- JOURNAL OF THE AMERICAN CHEMICAL SOCIETY, Band 87, 1965, Washington DC, USA; F. S. DOVELL et al: "Platinum metal sulfides as heterogeneous hydrogenation catalysts", Seiten 2767-2768

## Beschreibung

Gegenstand der vorliegenden Erfindung ist ein hinsichtlich Ökologie und Ökonomie verbessertes Verfahren zur Herstellung von N-Alkyl-halogenanilinen durch reduktive Alkylierung von Nitrohalogenbenzolen, insbesondere die Herstellung von N-Isopropyl-4-chloranilin durch reduktive Alkylierung von p-Nitrochlorbenzol mit Aceton in Gegenwart eines sulfitierten Platin-Katalysators von definierter, spezifischer Aktivität.

Das bei der katalytischen Hydrierung halogenhaltiger Nitroaromaten auftretende Problem der Halogenabspaltung ist auch bei reduktiven Alkylierungen halogenhaltiger Nitro- oder Aminoaromaten von besonderer Bedeutung, insbesondere bei technischen Prozessen.

Daher hat es nicht an Versuchen gefehlt, die bei der reduktiven Alkylierung von halogenhaltigen Nitroaromaten oder Anilinen auftretenden Nachteile, wie Halogenabspaltung, Kernhydrierung oder Mehrfachalkylierung zu vermeiden.

Der nächstliegende Stand der Technik zum erfindungsgemäßen Verfahren ist in DE-OS 2941070A1 beschrieben. Das dort beschriebene Verfahren ist dadurch charakterisiert, daß ein Halogenanilin oder Halogennitrobenzol mit 1 bis 10 Äquivalenten einer aliphatischen Carbonylverbindung in einem inerten Lösungsmittel, vorzugsweise aber in einer Lösung des Halogenanilins oder des Nitroaromaten in der aliphatischen Carbonylverbindung, unter Verwendung von 0,05 bis 2,0 Gew.-% eines sulfitierten Platin-Kohlenstoff-Katalysators bei Temperaturen von 70 bis 120°C, vorzugsweise bei 80 bis 100°C, umgesetzt wird. Der angewandte Wasserstoff-Überdruck beträgt nach diesem Verfahren 40 bis 100 bar. Die Ausbeuten an N-Halogenalkylanilinen betragen hierbei ca. 95 % der Theorie bei einem Reingehalt von meist über 98 %.

Halogenabspaltung wird nach diesem Verfahren "praktisch nicht beobachtet", jedoch sind keine analytischen Untersuchungen zur Halogenabspaltung angegeben.

Zum nächstliegenden Stand der Technik gehört ferner das in der US-PS 3 350 450 beschriebene Verfahren zur Herstellung aromatischer Amine durch katalytische Hydrierung von aromatischen Nitroverbindungen, insbesondere von N-Alkylhalogenanilinen durch reduktive Alkylierung von Halogenanilinen oder Halogennitrobenzolen in einem Ein- oder Zweistufenverfahren mit Wasserstoff und aliphatischen Aldehyden oder Ketonen in Gegenwart von Palladium-, Platin-, Rhodium-, Ruthenium- oder Kobalt-Sulfiden.

In der vorstehend genannten US-PS werden folgende Reaktionsbedingungen zur Herstellung von N-Isopropyl-4-chloranilin aus p-Nitrochlorbenzol und Aceton angegeben: Reaktionstemperatur 160 bis 200°C, Wasserstoffdruck ca. 70 bis 110 bar. Die Reaktionszeiten betragen je nach eingesetztem Metallsulfid-Katalysator 15 Minuten bis 6 1/2 Stunden. Die Ausbeuten an N-Alkylhalogenanilin betragen 92,5 bis 100 %.

Den beiden Verfahren des genannten nächstliegenden Standes der Technik ist gemeinsam, daß das eingesetzte Substrat (Halogen-Nitroaromat bzw. Halogenanilin) in einem 15 bis 10-molaren Überschuß an Carbonylverbindung umgesetzt wird. Aufgrund der angewandten drastischen Reaktionsbedingungen wird dabei ein erheblicher Teil der als Lösungsmittel eingesetzten Carbonylverbindung zum entsprechenden Alkohol hydriert. Dies bedeutet zum einen einen erhöhten Verbrauch an Wasserstoff, zum anderen läßt sich das entstehende Alkohol/Carbonylverbindungs-Gemisch nur destillativ mit hohem Energieeinsatz trennen. Zudem kann der entstandene Alkohol nicht wieder in den laufenden Prozeßzyklus eingeschleust werden und muß beispielsweise durch Einschleusung in einen anderen Prozeß entsorgt werden. Beide Verfahren haben zudem den Nachteil, daß sie bei relativ hohen Reaktionstemperaturen erfolgen müssen, um akzeptable Reaktionszeiten und damit Raum-Zeit-Ausbeuten zu erhalten.

Demgegenüber wurde nun gefunden, daß man N-Alkylhalogenaniline der allgemeinen Formel (I) 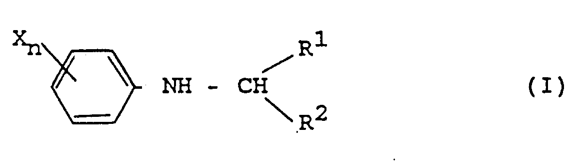
in welcher X ein Chlor- oder Bromatom und n die Zahl 1 oder 2 bedeuten, R¹ einen linearen oder verzweigten Alkyl (C₁-C₄)-Rest, R² einen linearen oder verzweigten Alkyl (C₁-C₆)-Rest darstellen oder R¹ und R² gemeinsam mit dem Kohlenstoffatom an dem sie sitzen einen fünf- oder sechsgliedrigen Cycloalkanring bilden können, unter Vermeidung der den genannten bekannten Verfahren anhaftenden Mängel vorteilhaft herstellen kann, indem man ein Halogennitrobenzol der allgemeinen Formel (II) 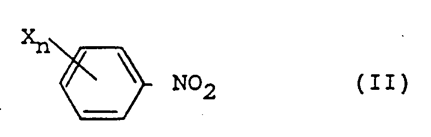
in welcher X und n die vorstehend genannten Bedeutungen haben, mit der 1- bis 1,5-fachen stöchiometrischen Menge einer Carbonylverbindung der allgemeinen Formel (III) 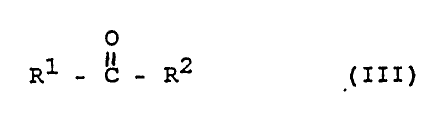
in welcher R¹ und R² die vorstehend genannten Bedeutungen haben oder R¹ und R² zusammen mit dem Kohlenstoffatom der Carbonylgruppe einen fünf- oder sechsgliedrigen Cycloalkanring bilden können, in einem gegenüber den Reaktionskomponenten unter den Reaktionsbedingungen inerten organischen Lösungsmittel bei Temperaturen von 30 bis 50°C , bei einem Wasserstoffüberdruck von 0 bis 50 bar, vorzugsweise 5 bis 25 bar, in Gegenwart eines sulfitierten Platin-Katalysators auf Aktivkohle umsetzt.

Als Halogennitrobenzole der genannten allgemeinen Formel II können beispielsweise p-Nitrochlorbenzol, o-Nitrochlorbenzol, p-Bromnitrobenzol, 2,5-Dichlornitrobenzol, 3,4-Dichlornitrobenzol, 3,5-Dichlornitrobenzol, 2,4-Dichlornitrobenzol oder 2,3-Dichlornitrobenzol eingesetzt werden.

Geeignete Carbonylverbindungen der genannten allgemeinen Formel III sind beispielsweise Aceton, Diethylketon, Methylethylketon, Methylisobutylketon, Methylisopropylketon, Ethylamylketon, Ethylisoamylketon, Cyclopentanon oder Cyclohexanon. Die Carbonylverbindungen werden in der ein- bis etwa 1,5-fachen stöchiometrischen Menge, bezogen auf Halogennitrobenzol, eingesetzt. Die Anwendung einer größeren Menge ist zwar möglich, bringt aber keinen zusätzlichen positiven Effekt, sondern macht das Verfahren zunehmend unwirtschaftlich.

Als inerte Lösungsmittel werden beim erfindungsgemäßen Verfahren beispielsweise Methanol, Ethanol, Isopropanol, Isobutanol, Ethylacetat, n-Butylacetat, Isopropylacetat, Isoamylalkohol oder 2-Ethylhexanol eingesetzt. Bevorzugt wird in Essigsäurealkylestern, wie Ethylacetat, n-Butylacetat oder Isopropylacetat umgesetzt.

Besondere Bedeutung kommt beim erfindungsgemäßen Verfahren dem eingesetzten Platin-Katalysator zu. Hierbei handelt es sich um einen käuflichen Katalysator (DEGUSSA Typ F1OP, 5 Gew.-% Platin auf Aktivkohle), der nach einem in DE-PS 2 105 780 beschriebenen Verfahren sulfitiert, d.h. desaktiviert wurde. Der Katalysator wie beim erfindungsgemäßen Verfahren in Mengen von etwa 2 bis etwa 10 Gewichtsprozent, vorzugsweise von etwa 3 bis etwa 5 Gewichtsprozent, bezogen auf Halogennitroverbindung, eingesetzt. Bei Reihenversuchen nach dem erfindungsgemäßen Verfahren wird der eingesetzte Katalysator bis zu 10mal ohne Ergänzung von Aufarbeitungsverlusten im Kreis geführt, wobei die Aktivität konstant bleibt.

Im einzelnen wird das erfindungsgemäße Verfahren zweckmäßigerweise wie folgt durchgeführt:
1 Mol eines halogenierten Nitrobenzols wird in einem inerten organischen Lösungsmittel, vorzugsweise einem Essigsäurealkylester, wie beispielsweise Ethylacetat oder insbesondere n-Butylacetat, an einem sulfitierten Platin-Katalysator in Anwesenheit eines Überschusses von etwa 20 Molprozent eines aliphatischen Ketons der Formel III bei maximal 50°C und einem Wasserstoffdruck von etwa 5 bis etwa 25 bar umgesetzt. Nach beendeter Reaktion wird der Platin-Katalystor abfiltriert und das eingesetzte Lösungsmittel im Vakuum abdestilliert. Der Rückstand wird im Vakuum fraktioniert. Die so erhaltenen N-Alkyl-halogenaniline haben in der Regel einen Reingehalt von > 99 %. Der eingesetzte Katalysator kann ohne Ausbeuteverluste im Kreis geführt werden.

Die Vorteile des erfindungsgemäßen Verfahrensliegen in einem geringeren Mengeneinsatz der aliphatischen Carbonylverbindung, in der wesentlich geringeren Reaktionstemperatur bei der reduktiven Alkylierung sowie in der nahezu unbegrenzten Rückführung des eingesetzten Lösungsmittels und Katalysators. Nach diesem Verfahren fällt außer dem Hydrier-Reaktionswasser keinerlei Abwasser an. Aufgrund des geringen CSB-Gehaltes ist das anfallende Hydrierwasser biologisch sehr gut abbaubar.

Beim erfindungsgemäßen Verfahren liegt die Halogenabspaltung bei 0 bis maximal 1 %, detektiert durch GC/MS-Verfahren. Bei höheren Reaktionstemperaturen als 55°C und ebenso bei höheren Wasserstoff-Drucken, wie beispielsweise 40 bis 60 bar, wurden zum Teil gravierend höhere Halogenabspaltungen beobachtet. Aus diesem Grund sind drastischere Reaktionsbedingungen als die angegebenen beim erfindungsgemäßen Verfahren eher nachteilig.

Prinzipiell läßt sich die reduktive Alkylierung auch mit den Halogenanilinen, die den Halogennitrobenzolen der Formel II entsprechen, durchführen. Diese Anilinderivate sind jedoch zum Teil aufgrund ihrer physiologischen Eigenschaften bedenklich und benötigen zu ihrer Herstellung aus den entsprechenden Nitroverbindungen einen zusätzlichen Verfahrensschritt.

Die verfahrensgemäß erhältlichen N-Alkyl-halogenaniline stellen wertvolle Vor- und Zwischenprodukte zur Herstellung von Pflanzenschutz- und Arzneimitteln sowie wertvolle Kupplungskomponenten zur Herstellung von Azofarbstoffen dar.

Die nachstehenden Beispiele sollen das erfindungsgemäße Verfahren näher erläutern, ohne es darauf zu beschränken.

### Beispiel 1

In einem 5 Liter V4A-Hydrierautoklav wurden 2500 ml n-Butylacetat und 397,7 g (2,5 Mol) p-Nitrochlorbenzol technisch rein und 175,7 g (3,0 Mol) Aceton bei Raumtemperatur vorgelegt. Nach Zugabe von 15,0 g sulfitiertem Platin-Katalysator (Wassergehalt des Katalysators ca. 50 %) wurde der Autoklav 3mal mit Stickstoff gespült und anschließend bei Raumtemperatur 10 bar Wasserstoff aufgedrückt. Danach wurde bei einer Temperatur < 50°C und bei einem Wasserstoffdruck von 5 bis 10 bar ca. 1 Stunde hydriert. Anschließend wurde der Wasserstoffdruck auf 25 bar erhöht und 6 Stunden bei 45 bis 50°C und einem Druck von 20 bis 25 bar nachgerührt.

Zur Aufarbeitung wurde der Autoklav nach Entleerung mit 150 ml n-Butylacetat ausgespült und der Katalysator über ein Filter abgetrennt und zweimal mit je 25 ml Butylacetat gewaschen. Der Katalysator wurde für den nächsten Ansatz, angefeuchtet mit 7,5 ml Wasser, aufbewahrt.

Das Filtrat wurde bei ca. 13 mbar aufdestilliert. Der Rückstand wurde anschließend im Vakuum bei 24 bis 27 mbar über eine Füllkörperkolonne destilliert. Man erhielt 372 g N-Isopropyl-4-chloranilin, das bei einem Siedepunkt von 122 bis 127°C überging.

Der Reingehalt des Produkts betrug 99,5 % (GC), was einer Ausbeute von 87 % der Theorie entspricht.

Die Chlorabspaltung, gaschromatographisch bestimmt über den Anteil n-Isopropylanilin, betrug weniger als 0,5 %. (Anmerkung: Im Rohprodukt wurden mittels GC/MS-Kopplung weitere 7 Nebenprodukte identifiziert, die allesamt jedoch im Spurenbereich < 0,1 % vorlagen.)

### Beispiel 2

Nach dem im Beispiel 1 beschriebenen Verfahren wurden 397,7 g (2,5 Mol) o-Nitrochlorbenzol umgesetzt. Die Ausbeute betrug 89 % der Theorie bei einem Reingehalt von 99,0 % (GC).

### Beispiel 3

Analog Beispiel 1 wurden 397,7 g (2,5 Mol) p-Nitrochlorbenzol in 2500 ml Ethylacetat umgesetzt. Die Aufarbeitung erfolgte prinzipiell analog, jedoch wurde das Lösungsmittel bei Normaldruck abdestilliert und der Rückstand anschließend ebenfalls im Vakuum fraktioniert.

### Beispiel 4

Analog Beispiel 1 wurden 397 g (2,5 Mol) o-Nitrochlorbenzol in 2500 ml Methanol umgesetzt. Nach der unter Beispiel 3 beschriebenen Aufarbeitung wurde N-Isopropyl-2-chloranilin in einer Ausbeute von 85 % der Theorie mit einem Reingehalt von 98,7 erhalten.

### Beispiel 5 (Vergleichsbeispiel)

Setzt man gemäß Beispiel 1 die dort beschriebenen Ausgangsprodukte und Lösungsmittel ein, führt die reduktive Alkylierung jedoch bei 100°C und 40 bis 60 bar Wasserstoffdruck durch, so erhält man 344,0 g N-Isopropyl-4-chloranilin, entsprechend einer Ausbeute von 70 % der Theorie. Daneben werden 56,7 g n-Isopropylanilin erhalten, was einer Chlorabspaltung von 14 % gleichkommt.

### Beispiel 6

480,0 g (2,5 Mol) 3,4-Dichlornitrobenzol wurden in 2500 ml Butylacetat unter den in Beispiel 1 beschriebenen Reaktionsbedingungen mit 10 g sulfitiertem Platin-Katalysator F1OP umgesetzt. Nach Abdestillieren des Lösungsmittels im Vakuum wurde der Rückstand fraktioniert. Man erhielt im Siedebereich 143 bis 148°C (13,33 bis 16 bar) 431,0 g N-Isopropyl-3,4-dichloranilin mit einem Reingehalt von 98,9 % (GC), was einer Ausbeute von 84,5 % der Theorie entspricht.

In J. Amer. Chem. Soc. 87, 2767-2768 (1965) wird die Eignung der Sulfide von Platinmetallen als Katalysator bei der Hydrierung von Nitrobenzol zu Anilin beschrieben. Ferner wird dort die weitere wichtige Anwendung der inredestehenden Katalysatoren bei der Reduktion von halogenhaltigen Nitroverbindungen zu Aminen, ohne Auftreten von Dehalogenierung, angegeben. Außerdem wird dort ausgeführt, daß die Sulfide von Platinmetallen zur reduktiven Alkylierung aliphatischer Amine bzw. deren Nitroalkanvorprodukte mit aliphatischen Ketonen eingesetzt werden können.

## Patentansprüche

1. Verfahren zur Herstellung von N-Alkyl-halogenanilinen der allgemeinen Formel (I) 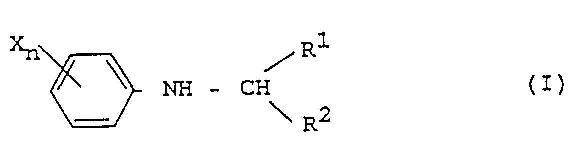 in welcher X ein Chlor- oder Bromatom und n die Zahl 1 oder 2 bedeuten, R¹ einen linearen oder verzweigten Alkyl-(C₁-C₄)-Rest, R² einen linearen oder verzweigten Alkyl-(C₁-C₆)-Rest darstellen oder R¹ und R² gemeinsam mit dem Kohlenstoffatom an dem sie sitzen einen fünf- oder sechsgliedrigen Cycloalkanring bilden können, dadurch gekennzeichnet, daß man ein Halogennitrobenzol der allgemeinen Formel (II) 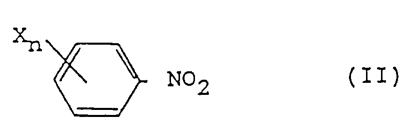 in welcher X und n die vorstehend genannten Bedeutungen haben, mit der 1- bis 1,5-fachen stöchiometrischen Menge einer Carbonylverbindung der allgemeinen Formel (III) 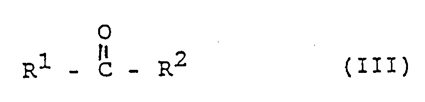 in welcher R¹ und R² die vorstehend genannten Bedeutungen haben oder R¹ und R² zusammen mit dem Kohlenstoffatom der Carbonylgruppe einen fünf- oder sechsgliedrigen Cycloalkanring bilden können, in einem gegenüber den Reaktionskomponenten unter den Reaktionsbedingungen inerten organischen Lösungsmittel bei Temperaturen von 30 bis 50°C, bei einem Wasserstoffüberdruck von 0 bis 50 bar, in Gegenwart eines sulfitierten Platin-Katalysators auf Aktivkohle umsetzt.

2. Verfahren nach Anspruch 1, dadurch gekennzeichnet, daß man bei einem Wasserstoffüberdruck von etwa 5 bis etwa 25 bar umsetzt.

3. Verfahren nach mindestens einem der Ansprüche 1 und 2, dadurch gekennzeichnet, daß man in Gegenwart von 2 bis 10 Gewichtsprozent Platinkatalysator auf Kohle, bezogen auf eingesetztes Halogennitrobenzol, umsetzt.

4. Verfahren nach mindestens einem der Ansprüche 1 bis 3, dadurch gekennzeichnet, daß man in Gegenwart von 3 bis 5 Gewichtsprozent Platinkatalysator auf Kohle, bezogen auf eingesetztes Halogennitrobenzol, umsetzt.

5. Verfahren nach mindestens einem der Ansprüche 1 bis 4, dadurch gekennzeichnet, daß man als Halogennitrobenzol p-Nitrochlorbenzol, o-Nitrochlorbenzol, p-Bromnitrobenzol, 2,5-Dichlornitrobenzol, 3,4-Dichlornitrobenzol, 3,5-Dichlornitrobenzol, 2,4-Dichlornitrobenzol oder 2,3-Dichlornitrobenzol einsetzt.

6. Verfahren nach mindestens einem der Ansprüche 1 bis 5, dadurch gekennzeichnet, daß man Aceton, Diethylketon, Methylethylketon, Methylisobutylketon, Methylisopropylketon, Ethylamylketon, Ethylisoamylketon, Cyclopentanon oder Cyclohexanon als Carbonylverbindung einsetzt.

7. Verfahren nach mindestens einem der Ansprüche 1 bis 6, dadurch gekennzeichnet, daß man in Methanol, Ethanol, Isopropanol, Isobutanol, Ethylacetat, n-Butylacetat, Isopropylacetat, Isoamylalkohol oder 2-Ethylhexanol als Lösungsmittel umsetzt.

## Claims

1. A process for the preparation of N-alkylhaloanilines of the formula (I) 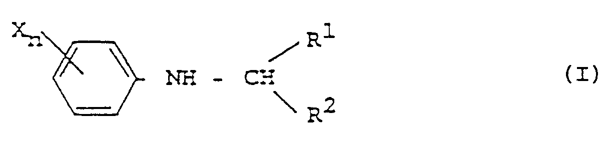 in which X is a chlorine or bromine atom and n is the number 1 or 2, R¹ is a linear or branched alkyl (C₁-C₄) radical, R² a linear or branched alkyl (C₁-C₆) radical or R¹ and R² together with the carbon atom to which they are bound can form a five- or six-membered cycloalkane ring, which comprises reacting a halonitrobenzene of the formula (II) 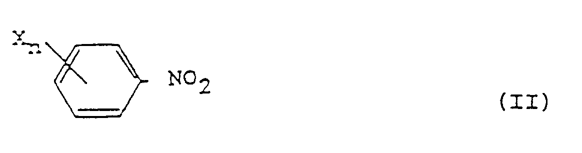 in which X and n have the abovementioned meanings with from 1 to 1.5 times the stoichiometric amount of a carbonyl compound of the formula (III) 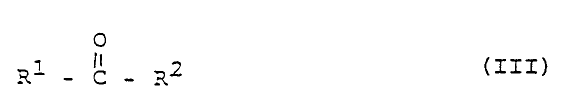 in which R¹ and R² have the abovementioned meanings or R¹ and R² together with the carbon atom of the carbonyl group can form a five- or six-membered cycloalkane ring, in an organic solvent which is inert towards the reactants under the reaction conditions at temperatures of from 30 to 50°C, at a hydrogen superatmospheric pressure of from 0 to 50 bar, in the presence of a sulfited platinum catalyst on activated carbon.

2. The process as claimed in Claim 1, wherein the reaction is carried out at a hydrogen superatmospheric pressure of about 5 to about 25 bar.

3. The process as claimed in at least one of claims 1 and 2, wherein the reaction is carried out in the presence of from 2 to 10 percent by weight of platinum catalyst on carbon, relative to the halonitrobenzene used.

4. The process as claimed in at least one of claims 1 to 3, wherein the reaction is carried out in the presence of from 3 to 5 percent by weight of platinum catalyst on carbon, relative to the halonitrobenzene used.

5. The process as claimed in at least one of claims 1 to 4, wherein the halonitrobenzene used is p-nitrochlorobenzene, o-nitrochlorobenzene, p-bromonitrobenzene, 2,5-dichloronitrobenzene, 3,4-dichloronitrobenzene, 3,5-dichloronitrobenzene, 2,4-dichloronitrobenzene or 2,3-dichloronitrobenzene.

6. The process as claimed in at least one of claims 1 to 5, wherein acetone, diethyl ketone, methyl ethyl ketone, methyl isobutyl ketone, methyl isopropyl ketone, ethyl amyl ketone, ethyl isoamyl ketone, cyclopentanone or cyclohexanone is used as the carbonyl compound.

7. The process as claimed in at least one of claims 1 to 6, wherein the reaction is carried out in methanol, ethanol, isopropanol, isobutanol, ethyl acetate, n-butyl acetate, isopropyl acetate, isoamyl alcohol or 2-ethylhexanol as the solvent.

## Revendications

1. Procédé pour la préparation de N-alkyl-halogénoanilines de formule générale (I) 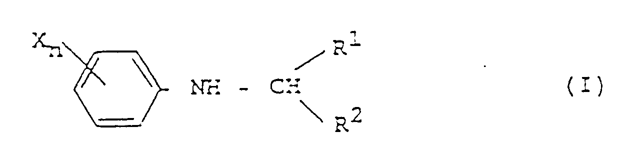 dans laquelle X représente un atome de chlore ou de brome et n représente le nombre 1 ou 2, R¹ représente un radical alkyle en C₁-C₄ linéaire ou ramifié, R² représente un radical alkyle en C₁-C₆ linéaire ou ramifié, ou R¹ et R² représentent ensemble avec l'atome de carbone sur lequel ils sont fixés un noyau cycloalcane à 5 ou 6 chaînons,
caractérisé en ce que l'on fait réagir un halogénonitrobenzène de formule générale (II) 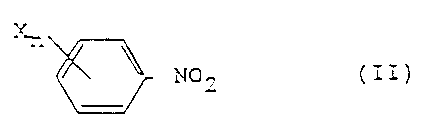 dans laquelle X et n ont les significations précitées, avec la quantité de 1 à 1,5 fois stoechiométrique d'un composé carbonyle de formule générale (III) 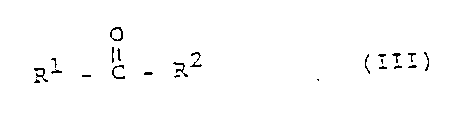 dans laquelle R¹ et R² ont les significations précitées, ou R¹ et R² représentent ensemble avec l'atome de carbone du groupe carbonyle un noyau cycloalcane à 5 ou 6 chaînons, dans un solvant organique inerte vis-à-vis des composants de la réaction dans les conditions de la réaction, à des températures de 30 à 50°C, avec une surpression d'hydrogène de 0 à 50 bars, en présence d'un catalyseur de platine sulfité sur du charbon actif.

2. Procédé selon la revendication 1, caractérisé en ce que l'on effectue la réaction avec une surpression d'hydrogène d'environ 5 à environ 25 bars.

3. Procédé selon au moins une des revendications 1 et 2, caractérisé en ce que l'on effectue la réaction en présence de 2 à 10 % en poids de catalyseur de platine sur du charbon, calculée sur l'halogénonitrobenzène utilisé.

4. Procédé selon au moins une des revendications 1 à 3, caractérisé en ce que l'on effectue la réaction en présence de 3 à 5 % en poids de catalyseur de platine sur du charbon, calculée sur l'halogénonitrobenzène utilisé.

5. Procédé selon au moins une des revendications 1 à 4, caractérisé en ce que l'on utilise comme halogénonitrobenzène le p-nitrochlorobenzène, le o-nitrochlorobenzène, le p-bromonitrobenzène, le 2,5-dichloronitrobenzène, le 3,4-dichloronitrobenzène, le 3,5-dichloronitrobenzène, le 2,4-dichloronitrobenzène ou le 2,3-dichloronitrobenzène.

6. Procédé selon au moins une des revendications 1 à 5, caractérisé en ce que l'on utilise comme composé carbonyle l'acétone, la diéthylcétone, la méthyléthylcétone, la méthylisobutylcétone, la méthylisopropylcétone, l'éthylamylcétone, l'éthylisoamylcétone, la cyclopentanone, ou la cyclohexanone.

7. Procédé selon au moins une des revendications 1 à 6, caractérisé en ce que l'on effectue la réaction dans le méthanol, l'éthanol, l'isopropanol, l'isobutanol, l'acétate d'éthyle, l'acétate de n-butyle, l'acétate d'isopropyle, l'alcool isoamylique ou le 2-éthylhexanol comme solvant.
